# EUROPÄISCHE PATENTSCHRIFT

(11) **EP 3 478 331 B1**
(45) Veröffentlichungstag und Bekanntmachung des Hinweises auf die Patenterteilung: **21.06.2023**
(21) Anmeldenummer: 17751229.0
(22) Anmeldetag: 28.06.2017
(51) Int. Cl.: A61L 27/56, A61L 27/18, A61L 27/24, A61L 27/50, A61L 27/58, A61F 2/28

(54) **BIOMATERIAL**
BIOMATERIAL
BIOMATÉRIAU

(30) Priorität: 30.06.2016 DE 102016007931
(43) Veröffentlichungstag der Anmeldung: 08.05.2019
(73) Patentinhaber: Matricel GmbH, 52134 Herzogenrath (DE); Charité - Universitätsmedizin Berlin, 10117 Berlin (DE)
(72) Erfinder: HESCHEL, Ingo, 52134 Herzogenrath (DE); LEEMHUIS, Hans, 52072 Aachen (DE); DUDA, Georg, 12209 Berlin (DE); HERRERA MARTÍN, Aarón Xerach, 10439 Berlin (DE); PETERSEN, Ansgar, 12045 Berlin (DE)
(74) Vertreter: Castell, Klaus
(86) Internationale Anmeldenummer: PCT/DE2017/000183
(87) Internationale Veröffentlichungsnummer: WO 2018/001401

(56) Entgegenhaltungen:
- EP-A1- 0 493 698
- EP-A1- 2 450 066
- WO-A2-2009/049650
- US-B2- 7 758 882
- WEISGERBER D W ET AL: "Evaluation of multi-scale mineralized collagen-polycaprolactone composites for bone tissue engineering", JOURNAL OF THE MECHANICAL BEHAVIOR OF BIOMEDICAL MATERIALS, ELSEVIER, AMSTERDAM, NL, Bd. 61, 6. April 2016 (2016-04-06), Seiten 318-327, XP029617782, ISSN: 1751-6161, DOI: 10.1016/J.JMBBM.2016.03.032 in der Anmeldung erwähnt

## Beschreibung

Die Erfindung betrifft ein Biomaterial. Dies ist ein synthetischer oder nichtlebender natürlicher Werkstoff, der in der Medizin für therapeutische oder diagnostische Zwecke eingesetzt wird und dabei in unmittelbaren Kontakt mit biologischem Gewebe des Körpers kommt.

Das Material tritt dabei in chemische, physikalische und biologische Wechselwirkungen mit den entsprechenden biologischen Systemen.

Dies schließt auch den kurzzeitigen Kontakt über die äußere Körperoberfläche, über Körperöffnungen und über von außen zugängliche Schleimhäute ein. Die Erfindung betrifft insbesondere solche Materialien, die zum längerfristigen Verbleib ins Körperinnere eingebracht werden.

Der Begriff Biomaterial bezieht sich dabei auf die stofflichen, insbesondere die chemischen und physikalischen, Eigenschaften des Materials. Kennzeichnend für ein Biomaterial ist eine aus seinen Eigenschaften resultierende Biokompatibilität, die sowohl die funktionale Ähnlichkeit zu körpereigenen Strukturen als auch eine angemessene biologische Verträglichkeit im Körper umfasst.

Viele Biomaterialien werden in Form von Implantaten im Körperinneren eingesetzt. Sie dienen dabei beispielsweise im Rahmen einer als Osteosynthese bezeichneten Behandlung zur Unterstützung der Heilung nach einem Knochenbruch ebenso wie dem dauerhaften Ersatz von Knochen, die durch einen Unfall oder durch Knochenkrebs irreparabel zerstört wurden. Möglich ist auch der Ersatz von Gelenkstrukturen, die durch chronische Erkrankungen oder langjährige Belastung abgenutzt sind. Mit Gefäßprothesen können Blutgefäße ersetzt werden, Stents dienen der Abstützung der Wand von Blutgefäßen. Beispiele für den Ersatz von Organteilen oder ganzen Organen durch Biomaterialien sind künstliche Herzklappen, künstliche Harnblasen oder Kunstherzen. Auch in der plastischen Chirurgie werden Biomaterialien eingesetzt, wie beispielsweise Glas für Kunstaugen, Silikon zur Brustrekonstruktion oder -vergrößerung oder Titan zur Schädelrekonstruktion.

Biomaterialien dienen jedoch auch der Unterstützung der Geweberegeneration, bei der das beschädigte oder fehlende Gewebe durch den Körper neu gebildet und/oder so umgebaut wird, dass es in seiner Funktion dem ursprünglichen intakten Gewebe gleichwertig ist oder ihm zumindest nahe kommt. In diesem Fall hat das Biomaterial keine dauerhafte sondern nur eine zeitlich beschränkte Funktion und wird idealerweise im Laufe der Zeit im Körper abgebaut bis es vollständig verschwunden und durch das neugebildete Gewebe ersetzt ist. Als Beispiele sind die Regeneration von Knochen, Haut, Nerven, Muskeln, Sehnen und Bändern, Bindegewebe, Knorpel, Speiseröhre, Stimmband, Herzmuskel, Herzklappen zu nennen. Die Biomaterialien, die hierbei zum Einsatz kommen, müssen daher weitere Eigenschaften aufweisen, da sie insbesondere zumindest zum Teil durch im Körper ablaufende Prozesse abgebaut bzw. remodelliert werden müssen, wobei auch die Abbauprodukte biokompatibel sein müssen. Solche "bioresorbierbaren" Biomaterialien können aus natürlichen Materialien autogenen, allogenen oder xenogenen oder auch pflanzlichen Ursprungs bestehen (wie z.B. aus Kollagen, Elastin, Fibrin, Hydroxylapatit, Chitosan, Alginat) oder aus synthetischen Materialien (wie z.B. PLA, PGA, PCL, PLGA).

In der regenerativen Medizin wird die vollständige Wiederherstellung der Funktion von beschädigtem, erkranktem oder verlorengegangenem Gewebe (z.B. durch Trauma) angestrebt. Die regenerative Medizin befasst sich sowohl mit dem sogenannten "Tissue Engineering", dem Züchten von Ersatzgewebe mit Eigenschaften, welche dem zu ersetzenden Gewebe identisch oder möglichst ähnlich sind, aber auch mit der Wiederherstellung der Funktion unter Ausnutzung körpereigener, sogenannter "endogener" Regenerationsmechanismen und -prinzipien. Endogene Regenerationsmechanismen können z.B. durch das Einbringen von Biomaterialien, Zytokinen (z.B. Wachstumsfaktoren) oder Zellen oder einer Kombination aus diesen unterstützt werden. Insofern das Biomaterial, z.B. durch seine mechanischen, physikalischen oder chemischen Eigenschaften, in der Lage ist Zellen aus dem umliegenden Gewebe zu rekrutieren und im endogenen Regenerationsprozess zu unterstützen und zu leiten, ist auch ein reiner Biomaterialansatz denkbar, bei dem von außen keine Zellen oder Zytokine in den Körper eingebracht werden müssen.

Das Prinzip endogene Regenerationsprozesse zur Geweberegeneration zu nutzen hat unter Einsatz bestehender Biomaterialansätze zu widersprüchlichen Ergebnissen bezüglich der Qualität des resultierenden Gewebes geführt. Die mangelnde Qualität des Regenerats und ein damit verbundener unklarer Langzeiterfolg führen dazu, dass sich bisher wenige bzw. keines dieser vorgefertigten und gebrauchsfertigen "off the shelf" Materialien oder Materialkombinationen zur Geweberegeneration in der Klinik durchsetzen konnte.

In sogenannten Tissue-Engineering-Ansätzen soll der möglichst exakte Nachbau des intakten Gewebes eine schnelle Integration des Biomaterials und eine Wiederherstellung der Gewebefunktion gewährleisten. Allerdings ist die Komplexität des nativen Gewebes bisher nicht vollständig abbildbar, sodass entsprechende Tissue-Engineering Produkte bislang nicht mit dem nativen autologen Gewebe vergleichbar sind.

In "Weisgerber DW, Erning K, Flanagan CL, Hollister SJ, Harley BAC. Evaluation of multiscale mineralized collagen-polycaprolactone composites for bone tissue engineering. J Mech Behav Biomed Mater. 2016 Aug;61:318-327" wird ein PCL-Traggerüst, das ein Kollagen-Glycosaminoglycan-Gerüst umgibt offenbart.

Die Aufgabe der Erfindung liegt darin, ein Material zu finden, das es erleichtert nach einer Struktur- und/oder Funktionsveränderung innerhalb des Organismus die ursprüngliche Situation wieder herzustellen oder eine angestrebte Situation zu erreichen.

Diese Aufgabe wird mit einem Biomaterial mit den Merkmalen des Patentanspruchs 1 gelöst.

Vorteilhafte Ausgestaltungen sind Gegenstand der Unteransprüche.

Um die Materialanteile bestimmen zu können, wenn Materialqualitäten als fließender Übergang ineinander übergehen oder wenn die Steifheit bei ähnlichem Material im Wesentlichen durch die Struktur bestimmt wird, wird festgelegt, dass der erste Materialanteil derjenige Anteil ist, der eine geringere Steifheit hat als die mittlere Steifheit des Biomaterials. Die mittlere Steifheit wird auf das Volumen bezogen ermittelt.

Unter dem Volumenanteil des steiferen Materials wird das tatsächliche Volumen des zweiten Materialanteils unter Ausschluss der Hohlräume im Verhältnis zum Gesamtvolumen des Biomaterials verstanden. Dabei ist das Gesamtvolumen jeweils das Volumen der Masse plus das Volumen der von der Masse umschlossenen Hohlräume. Das heißt ein poröser Quader hat das gleiche Gesamtvolumen wie ein gleichgroßes Vollmaterial.

Studien z.B. von Engler et.al, Harley et. al., Park et al. und Reilly et. al. haben gezeigt, dass die Stammzellmigration und Differenzierung entscheidend durch die Eigenschaften Steifigkeit und Struktur der Mikroumgebung der Zelle beeinflusst werden.

Weitere Untersuchungen in 3D Hydrogelen bestätigten, dass eine mechanische Steifigkeit mit einem Elastizitätsmodul zwischen ca. 10 und 100 kPa die osteogene Differenzierung von mesenchymalen Stammzellen (MSCs) induzieren, während geringere Steifigkeiten zu einer adipogenen Differenzierung führen.

Experimentelle Untersuchungen und Computermodelle haben gezeigt, dass neben der Substratsteifigkeit auch die lokale mechanische Dehnung bzw. Stauchung ein entscheidender Einflussfaktor für die Differenzierung von Zellen- und Gewebe bei der Knochenregeneration darstellt Nachfolgend soll der Begriff "Stauchung" auch unter dem Oberbegriff "Dehnung" subsummiert werden, so dass die bezifferten Dehnungen auch Verkürzungen um den betragsmäßigen Prozentsatz beinhalten sollen. Zu hohe Dehnungen (ε > 30 %) behindern die Regeneration, mittlere Dehnungen (ε = ca. 4 bis 12 %) unterstützen die Bildung von Knorpel, geringe Dehnungen (ε = ca. 0,04 bis 4 %) die Mineralisation von Knorpel und die Formation von Knochen.

Bei der Erfindung soll die Mechanik des Materials einerseits zur Stabilisierung des weicheren Materialanteils während des Regenerationsprozesses und andererseits als Regulator der Zellfunktionen und der Gewebedifferenzierung genutzt werden. Über die räumlich spezifische Gestaltung der Komponenten werden die auf das Material wirkenden Kräfte in die für die jeweilige Differenzierung notwendige Dehnung übersetzt. Durch die Struktur soll das Material außerdem eine Aufspann-Funktion wahrnehmen, die einen Kollaps der weicheren Biomaterialstruktur während des Heilungsprozesses verhindern soll.

Eine Ausführungsform sieht ein Mehrkomponenten-Biomaterial vor, das aus mindestens zwei Komponenten besteht, die sich in erster Linie in ihren mechanischen und strukturellen Eigenschaften unterscheiden. Das im Weiteren als "Mechanohybridscaffold" bezeichnete Biomaterial ist mechanisch und strukturell hierarchisch aufgebaut. Bei dem Zwei-Komponenten-Biomaterial dient die Komponente mit geringerer Steifigkeit und geringerer Strukturgröße (erster Materialanteil) als Trägermaterial für die Zellen, die die Regeneration bewirken sollen (lokalen Ursprungs bzw. gewonnen und/oder kultiviert) und als zell-steuerndes Material; die Komponente mit höherer Struktursteifigkeit und Strukturgröße (zweiter Materialanteil) dient als mechanisch stabilisierendes Element zur Realisierung der gewünschten makroskopischen Eigenschaften. Hierbei ist als Steifigkeit die Struktursteifigkeit gemeint. Diese wird von den Eigenschaften der verwendeten Materialien, deren Struktur und von der geometrischen Anordnung der Komponenten bei Mehrkomponentenmaterialien bestimmt. Der erste Materialanteil ist in den zweiten Materialanteil formschlüssig strukturell integriert. Mechanische Kräfte, die auf den zweiten Materialanteil wirken, werden somit auf den ersten Materialanteil vorzugsweise sowohl über einen Formschluss als auch bei Adhäsionskräften zwischen den beiden Materialkomponenten über Kraftschluss übertragen, wobei die Steifigkeit bei einer entsprechenden mechanischen Belastung die von dem zweiten Materialanteil in dem ersten Materialanteil wirkende Dehnung bestimmt. Mit dem Begriff "Scaffold" werden dabei "Gerüststrukturen" bezeichnet, die im Verlauf der Regeneration durch körpereigene Prozesse teilweise oder ganz remodelliert werden können.

Die Funktion der Strukturierung des Mechanohybridscaffolds ist es, in erster Linie den Regenerationsprozess entlang einer vorgegebenen Richtung zu leiten. Dieses Prinzip wurde bereits erfolgreich bei der Regeneration von z. B. Haut, Weichgewebe und peripheren Nerven (US6447701 (B1) - Method for producing porous structures) und von den Erfindern bei der Regeneration von Knochen eingesetzt. Ein strukturierter und gerichteter Regenerationsprozess ist erwartungsgemäß jedoch auch für die Regeneration vieler weiterer Gewebe (z.B. Muskel, Sehnen, Bänder, Gefäße, röhrenförmige Hohlorgane) von Bedeutung. Entsprechend weist zumeist mindestens einer der Materialanteile eine strukturelle Anisotropie auf. Der strukturell hierarchische Aufbau gewährleistet dabei auch, dass die Architektur und die lokale mechanische Steifigkeit des ersten Materialanteils durch den zweiten möglichst wenig beeinflusst wird.

In eigenen Tierstudien wurde belegt, dass bereits der erste Materialanteil alleine bei geeigneter Wahl der Materialparameter (Steifigkeit, Porenarchitektur, Resorbierbarkeit) den Knochenregenerationsprozess in einem Knochendefekt unterstützen kann. In den Studien wurde ein Knochenwachstum entlang der Scaffoldporen über den Prozess der enchondralen Ossifikation beobachtet, während beim gleichen Tiermodell ohne Scaffold eine direkte - sogenannte intramembranöse - oberflächliche Knochenformation und eine geringere volumetrische Knochenneubildung beobachtet wurde. Bei der enchondralen Ossifikation wird Knochen über die Mineralisation einer Knorpelphase gebildet, wie es auch in der Wachstumsfuge bei Kindern und Jugendlichen beobachtet wird. Im Gegensatz hierzu wird bei der direkten intramembranösen Knochenformation Knochengewebe direkt auf bestehendem Knochen ohne Knorpelphase abgelegt. Es wurde jedoch auch beobachtet, dass bei diesem Ansatz der weiche, für die Induktion mechanisch und strukturell optimierte, erste Materialanteil im Körper durch Zellkräfte und Kräfte von umliegendem Gewebe verformt und seine Porenarchitektur beeinträchtigt wird und seine Leitfunktion verliert. Es ist daher notwendig den ersten Materialanteil mit einem zweiten mechanisch stabilisierenden Materialanteil zu kombinieren, um das Regenerationspotential des Materials aufrecht zu erhalten bzw. weiter zu erhöhen. Bei einem zweiten mechanisch stabilisierenden Materialanteil kann zudem die Steifigkeit des ersten Materialanteils noch weiter reduziert werden, so dass noch größere - für die Geweberegeneration bestimmter Gewebe vorteilhaftere Dehnungen durch die Zellen erzielt werden können, ohne dass die Gesamtstruktur des Mechanohybridscaffolds kollabiert.

Vorteilhaft ist es, wenn mindestens ein Materialanteil und vorzugsweise beide Materialanteile gerichtete Strukturen aufweisen. Eine Struktur ist gerichtet, wenn sich der größte und der kleinste Abstand zwischen Strukturelementen (Porenwänden, Stegen, etc.) der entlang aller Raumrichtungen zu finden ist um mindestens das Dreifache unterscheidet. Die Richtung mit dem größten Abstand der Strukturelemente entspricht der bevorzugten Ausrichtung des Materials bzw. der Richtung der strukturellen Anisotropie.

In einer möglichen Realisierung sind einer oder beide Materialanteile bioabbaubar. In diesem Fall ist die Resorbierbarkeit auf die Geschwindigkeit des Regenerationsprozesses und die ablaufenden Gewebeumbauprozesse abgestimmt. Beide Materialanteile sind miteinander soweit form- oder kraftschlüssig verbunden, dass mechanische Signale von dem einen zu dem anderen Materialanteil übertragen werden.

Die anisotrope Strukturierung des ersten und/oder zweiten Materialanteils erlaubt zusätzlich die Realisierung unterschiedlicher mechanischer Steifigkeiten in unterschiedliche Raumrichtungen (anisotrope Steifigkeit). Dies ermöglicht es, den Mechanohybridscaffold so zu gestalten, dass er z.B. senkrecht zur lasttragenden Richtung leicht komprimierbar ist, was große Vorteile beim Einbringen mittels minimalinvasiven Operationsmethoden (z.B. Endoskopie) bietet. Elastische Materialeigenschaften gewährleisten das Wiederaufspannen von beiden Materialanteilen nach minimal-invasivem Einbringen.

Es wird daher vorgeschlagen, dass mindestens ein Materialanteil und vorzugsweise sogar beide Materialanteile elastisch verformbar sind. Der Bereich der elastischen Verformbarkeit in einer Raumrichtung liegt in der Praxis bei einer Kompression auf minimal die 0,1-fache Länge bzw. bei Dehnung auf maximal die 10-fache Länge.

Der erste Materialanteil kann aus mehreren Materialien oder Komponenten bestehen, die z.B. in Form eines Bi- oder Multilayerscaffolds angeordnet sind. Diese können sich im Material (z.B. Hydrogel, Kollagen, Chitosan, usw.) oder in den Materialparametern (z.B. Feststoffgehalt, Dichte, Porosität, etc.) unterscheiden. Ebenso kann der zweite Materialanteil aus mehreren Materialien oder Komponenten bestehen und als Bi- oder Multilayerscaffold aufgebaut sein. Die mechanischen Eigenschaften der Materialanteile können räumlich unterschiedlich gestaltet sein, entweder als diskrete Lagen bzw. Schichten oder mit fließendem (graduellem) Übergang oder mit einem weichen oder festen Kern. Eine solche Gestaltung kann z.B. genutzt werden, um unterschiedliche Dehnungen in unterschiedlichen Bereichen des Materials zu realisieren. Hierdurch können z.B. Übergänge zwischen unterschiedlichen Geweben nachgebildet werden (z.B. Muskel-Sehne, Sehne-Knochen, Knorpel-Knochen). Die räumlichen Unterschiede in z.B. Dehnung, Porengröße oder Steifigkeit können zur Differenzierung des regenerierenden Gewebes in unterschiedliche Gewebetypen innerhalb eines Materials genutzt werden.

Gemäß der beanspruchten Erfindung sind die ersten und zweiten Materialanteile strukturell miteinander verwoben und nicht als diskrete Lagen gestaltet. Die Stützstruktur ist wie bei einem Endoskelett integraler und vom Scaffold umgebener Teil des Biomaterials. (Def aus Wikipedia: Als *Endoskelett* (auch Innenskelett genannt) bezeichnet man in der Biologie eine mechanische Stützstruktur (Skelett), die nicht Teil der äußeren Hülle des Organismus ist.)

Das hierarchisch strukturierte Biomaterial kann zusätzlich weitere Komponenten in Form von Wachstumsfaktoren, bioaktiven Partikeln oder Zusatzstoffen, die die Bioaktivität des Materials modifizieren, beinhalten. Die Zusatzfaktoren können im ersten Materialanteil und/oder dem zweiten Materialanteil integriert oder in die poröse Struktur eingebracht sein.

Das hierarchisch strukturierte Biomaterial kann zusätzlich vor dem Einbringen in den Körper mit Zellen kombiniert werden, welche den Regenerationsprozess unterstützen. Diese Zellen können entweder während des Eingriffs gewonnen werden (z.B. aus Gewebe oder Blut) oder in einer vorausgehenden Prozedur entnommen und ggf. konserviert und/oder in vitro expandiert worden sein.

Die Erfindung hat gegenüber bekannten Scaffolds mehrere Vorteile. Die Leitstruktur bedingt eine strukturelle und/oder mechanische Anisotropie mindestens eines der Anteile zur Ausrichtung des Geweberegenerationsprozesses in Geweben mit spezifischen strukturellen Eigenschaften wie beispielsweise von Knochen, Bändern, Muskeln und/oder Knorpel. Bimodale mechanische Eigenschaften bedingen eine auf zellulärer Ebene mikroskopisch weiche und makroskopisch lasttragende Funktion zur Stabilisierung der Scaffoldintegrität.

Ein hierarchischer Aufbau ermöglicht eine optimierte Mechanik und Geometrie auf unterschiedlichen Längenskalen bei gleichem Bauprinzip, der zu speziellen mechanischen und geometrischen Eigenschaften führt und ermöglicht außerdem auch die nahtlose Übertragung von mechanischen Signalen zwischen den Komponenten. Der Aufbau aus unterschiedlichen Materialanteilen ermöglicht räumlich einstellbare mechanische Eigenschaften. Durch eine Aufspannfunktion der Stützstruktur wird eine mechanische Langzeitstabilität, das heißt eine Formstabilität von bioabbaubaren Scaffolds erreicht. Sofern mindestens der zweite Materialanteil eine Komprimierbarkeit und Elastizität aufweist, ergibt sich ein Scaffold, das besonders für den Einsatz bei minimalinvasiven Operationstechniken geeignet ist. Dabei kann eine mechanische Anisotropie (sehr fest in mechanischer Funktionsrichtung, leicht komprimierbar senkrecht dazu) genutzt werden. Der zweite Materialanteil spannt dabei den ersten Materialanteil nach Kompression wieder auf. Einzelne Komponenten oder Materialanteile können auf die jeweilige Aufgabe mechanisch, strukturell und ggf. biochemisch optimiert werden. Dabei kann der erste Materialanteil zur Steuerung von zellulären Prozessen optimiert werden und der zweite Materialanteil zur Stabilisierung der primären Struktur und zur Übertragung von biomechanischen Signalen an den ersten Materialanteil. Dies ermöglicht eine diskrete Verteilung oder einen kontinuierlichen Übergang der mechanischen Eigenschaften des Materials. Durch die Kombination oder Integration von Partikeln oder Zusatzstoffen/-wirkstoffen können mechanische, chemische, biologische und physikalische Eigenschaften erweitert werden. Außerdem erschließt sich die Möglichkeit der Zellbesiedlung.

Folgende Materialien können beispielhaft für die Realisierung der beiden Komponenten zum Einsatz kommen: Für den ersten Materialanteil kommen prinzipiell alle weichen und degradierbaren, biokompatiblen Materialien einzeln oder in Kombination in Frage. Dies sind beispielsweise Kollagen, Fibrin, Hyaluronsäure, Elastin, Alginat, Polyethylenglykol (PEG), Polyvinylalkohol (PVA), Polysaccharide, Chitosan, Seide und andere Materialien. Für den zweiten Materialanteil kommen prinzipiell alle degradierbaren und nicht-degradierbaren biokompatiblen Materialien in Frage. Dies sind beispielsweise Polyamid (PA), Polycaprolacton (PCL), Polylactid-co-Glycolid (PLGA), Polylactid (PLA), Polyethylenglykol (PEG), Polyglycolid (PGA), Polyvinylalkohol (PVA), Polyetheretherketon (PEEK), Polymethylmethacrylat (PMMA), Metalle (z.B. Nitinol, chirurgische Stähle, Titan- und Titanlegierungen, Magnesium), Keramiken und Keramik-Komposite und viele andere Materialien.

Verschiedene Materialparameter können beispielhaft für die Realisierung der beiden Komponenten zum Einsatz kommen. Im Hinblick auf die Struktur wird für den ersten Materialanteil eine hochporöse Struktur mit einer Porosität > 70 % und eine offenporöse sphärische, kanalförmige oder komplexere Geometrie vorgeschlagen. Für den zweiten Materialanteil wird eine Gerüststruktur mit möglichst geringem Materialvolumen bzw. hoher Porosität (> 70%) vorgeschlagen, die beispielsweise aus polygonförmigen Elementen (Wabenstruktur), Kanälen oder sphärischen Geometrien aufgebaut ist. Die Poren bzw. Strukturgrößen können für den ersten Materialanteil zwischen 10 - 1000 µm und für den zweiten Materialanteil zwischen 100 µm - 10 mm liegen. Die Steifigkeiten können für den ersten Materialanteil bei E < 1 MPa und für den zweiten Materialanteil bei E > 100 kPa liegen. Für den ersten Materialanteil wird ein Material vorgeschlagen, das nach zyklischer Kompression und Entspannung seine Form (Porengeometrie) in Kombination mit dem zweiten Materialanteil nicht wesentlich verändert und für den zweiten Materialanteil ein elastisches, weitgehend reversibel verformbares Material mit nur geringer oder keiner Energiedissipation bei einer zyklischen Kompression und Entspannung.

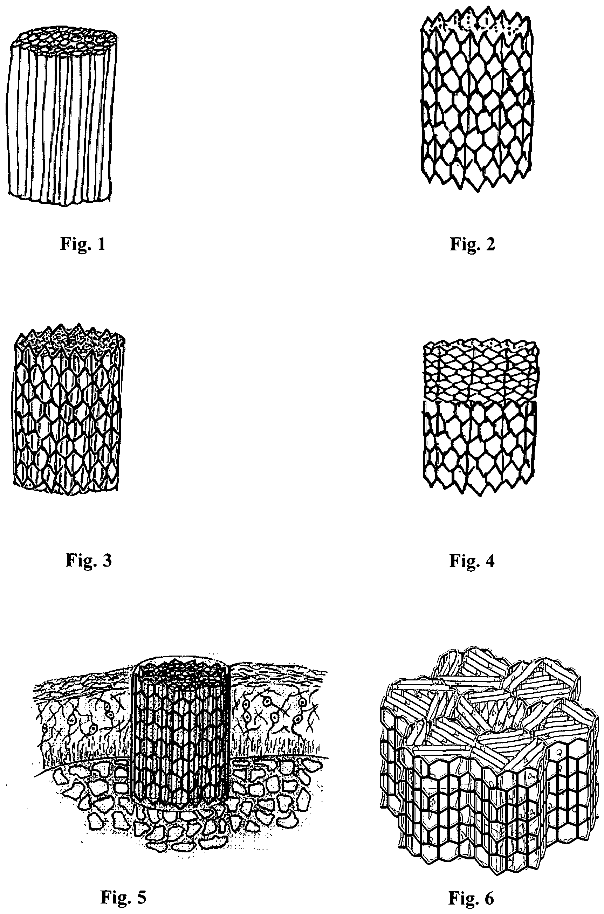

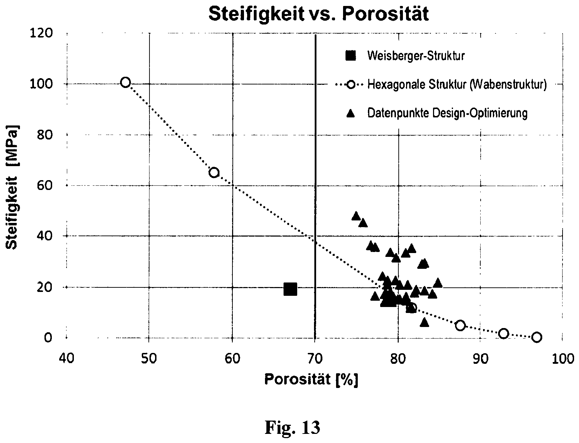

Allgemein ist bekannt, dass die Porosität eines Biomaterials zur Nutzung endogener Regenrationsprozesse für die Gewebeheilung möglichst hoch sein sollte. Eine hohe Porosität ermöglicht eine bessere Versorgung der Zellen mittels Diffusion und Konvektion von gelösten Stoffen (z.B. Zufuhr von Nährstoffen und Sauerstoff, Abfuhr von Stoffwechselprodukten und Gasen). Außerdem ist eine hohe Porosität notwendig, um die endogenen Gewebeprozesse durch das Biomaterial räumlich nicht zu behindern. Da andererseits die äußere mechanische Steifigkeit des in der Erfindung beschriebenen Biomaterials für die Funktionalität des ersten Materialanteils essentiell ist, wird die Architektur des zweiten Materialanteils so gewählt, dass seine Struktursteifigkeit im Vergleich zu dem von ihm eingenommenen Volumenanteil möglichst hoch ist. Dies ist nur durch einen entsprechenden Designprozess unter Berücksichtigung ingenieurswissenschaftlicher Prinzipien möglich. Eigene Computersimulationen mittels Finite Elemente Methode (FEM) haben gezeigt, dass durch einen solchen Prozess die Struktursteifigkeit des zweiten Materialanteils bezogen auf die Steifigkeit des eingesetzten Materials deutlich erhöht werden kann. Erst dadurch sind unter Berücksichtigung der notwendigen Steifigkeit des Biomaterials, z.B. für den Einsatz in Knochendefekten, Porositäten von >70 Volumenprozent (70 % (v/v) erreichbar. Durch das Design der zweiten Materialkomponente können die in Figur 13 angegebenen Steifigkeiten erreicht oder überschritten werden (Struktur entsprechend Ausführungsbeispiel in Fig. 6). Zwischenwerte sind durch Interpolation zu berechnen. Stiffness ist Struktursteifigkeit in MPa und Porosity ist Porosität in Volumenprozent. Der Stand der Technik (Weisberger et al., 2016) zeigt bei einer Porosität von 67 Volumenprozent lediglich eine relative Struktursteifigkeit von 6.3 %, der Wert einer designoptimierten Struktur liegt bei >15 %. Die relative Struktursteifigkeit ist dabei die Struktursteifigkeit geteilt durch die Materialsteifigkeit).

In einer Publikation zeigten Weisberger et al. (Journal of the Mechanical Behavior of Biomedical Materials 2016, Volume 61, Pages 318-327, DOI: 10.1016/j.jmbbm.2016.03.032), dass die Steifigkeit eines weichen Biomaterials (Mineralisierter Kollagen/Glycosaminoglycan Scaffold) durch Kombination mit einer gedruckten Stabilisierungsstruktur (Polycaprolacton, PCL) signifikant erhöht werden kann. Allerdings ist die Porosität der Stabilisierungsstruktur gering (67 Volumenprozent), sodass mit einer deutlichen Beeinträchtigung endogener Regenerationsprozesse bei Implantation zu rechnen ist. Außerdem weisen beide Materialkomponenten keine gerichteten Strukturen auf und können damit den Regenerationsprozess nicht entlang definierter Richtungen leiten, was für die Regeneration muskuloskeletaler Gewebe und anderer Gewebe mit gerichteten Strukturen (z.B. Nerven) von Nachteil ist. Ziel der Erfindung ist es, eine strukturell und mechanisch optimierte Umgebung für die Geweberegeneration zu schaffen. Hierfür sind Porositäten mit Werten >70 Volumenprozent, besonders bevorzugt >80 Volumenprozent notwendig. Für resorbierbare Materialien lassen sich solche Porositäten unter Erhalt der mechanischen Struktursteifigkeit nur durch eine auf die mechanische Funktion abgestimmte Materialarchitektur erreichen. Hierbei wird die Materialmenge so verteilt, dass sich in der gewünschten Raumrichtung eine möglichst hohe strukturelle Steifigkeit ergibt. Die Identifikation einer optimalen Architektur ist z.B. unter Nutzung von "Finite Elemente"-Computersimulationen möglich.

Weiterhin weist die von Weisberger vorgeschlagene Stabilisierungsstruktur innerhalb der Einheitszelle ("Unit cell") stark unterschiedliche Materialstärken/Materialdurchmesser auf. Im Zuge der Materialdegradation nach Implantation kommt es hierdurch zu einer inhomogenen Resorption. Bereiche geringen Materialdurchmessers werden schneller abgebaut und führen zum mechanischen Versagen der Struktur. Bereiche großen Materialdurchmessers führen im Zuge der Degradation über einen längeren Zeitraum zu einer Gewebereaktion ohne eine mechanische Funktion zu erfüllen. Aus diesem Grund ist ein möglichst einheitlicher Durchmesser der Strukturelemente innerhalb einer Einheitszelle von Vorteil.

Besonders vorteilhaft ist, wenn der erste Materialanteil aus Kollagen besteht und im Hinblick auf Feststoffgehalt, Porengröße und Abbauzeit optimiert wird. Eine derartige Ausführungsform konnte in einem Knochendefekt kritischer Größe ein Knochenwachstum über eine Knorpelphase (enchondrale Ossifikation) induzieren.

Das Material kann in unterschiedlichen Bereichen der Gewebedefektregeneration eingesetzt werden. Eine besonders relevante Anwendung liegt in der Wirbelkörperfusion, der Regeneration von größeren Defekten der langen Röhrenknochen und bei der Regeneration von osteochondralen Defekten z.B. im Kniegelenk. Über diese Anwendungen hinaus ist der Einsatz des Materials bei der Regeneration von Sehnen, Sehnen-Muskel und Sehnen-Knochen Übergängen nach Ruptur zu erwähnen. Dazu können aus den Biomaterial beispielsweise Knochenregenerationsmaterialien zur Heilung von Knochendefekten z.B. in Tibia oder Radius, Knochenregenerationsmaterial zur Fusion von Wirbelkörpern, Material zur Regeneration von osteochondralen Defekten z.B. nach Trauma oder Osteochondrosis dissecans, Material zur Regeneration von Hohlorganen (Gefäße, Speiseröhre etc....), Material zur Regeneration von Stimmbändern, Material zur Regeneration von Haut, Material zur Regeneration von Herzklappen und Herzmuskel und Material zur Regeneration von peripheren Nerven und des zentralen Nervensystems (Rückenmark) hergestellt werden.

Die Struktur beider Materialkomponenten kann aus einfachen Grundgeometrien/ Grundstrukturen aufgebaut sein. Eine solche Grundstruktur kann, analog zur Kristallographie, als Einheitszelle beschrieben werden, aus der die Gesamtstruktur durch Verschiebung und/oder Rotation in bestimmten Raumrichtungen oder entlang bestimmter Achsen entsteht. Hierdurch entsteht ein repetitives Muster mit Struktursymmetrien in Verschiebung und Rotation.

Besonders vorteilhaft ist, wenn der zweite Materialanteil Versteifungsstrukturen aufweist. Das Volumen oder die Masse des zweiten Materialanteils kann so verteilt sein, dass sich eine möglichst hohe mechanische Steifigkeit oder Festigkeit (oder auch Dehnbarkeit) bei möglichst geringem Materialvolumen bzw. bei möglichst hoher Porosität ergibt. Dies kann z.B. durch eine geeignete Anordnung von dünnen Materialstegen oder Wänden in bestimmten Raumrichtungen erreicht werden (=Leichtbauprinzip). Diese Strukturen können beispielsweise wie Bienenwaben aufgebaut sein.

Besonders vorteilhaft ist es, wenn die Komponente mindestens eines Materialanteils und vorzugsweise beider Materialanteile Stoffe, Elemente oder Strukturen enthält, welche mittels in der Klinik eingesetzter Bildgebender Verfahren darstellbar sind, so dass die Ausrichtung und Positionierung des Biomaterials mit Hilfe von Röntgenaufnahmen, Aufnahmen im Computertomographen oder durch Magnetresonanztomographie überprüft und der Heilungsprozess kontrolliert werden kann.

Besonders vorteilhaft ist es, wenn der erste oder zweite Materialanteil durch einen externen Stimulus (z.B. Kraft, Temperatur, elektrische oder magnetische Felder) in seiner Form verändert werden kann, um z.B. zyklische Verformungen zu induzieren, die mechanobiologische Auswirkungen auf die regenerierenden Zellen haben. Die getriggerte Verformung des zweiten Materialanteils kann die minimal invasive Implantation des Biomaterials vereinfachen. Im Idealfall erfolgt die externe Stimulation nicht invasiv, d.h. sie gelingt (z.B. durch das magnetische Feld) ohne Berührung des Biomaterials. Eine zyklische Verformung des Biomaterials kann für die gezielte Differenzierung der Zellen und die Art und Orientierung der von den Zellen gebildeten extrazellulären Matrix von Vorteil sein.

Biologic-free mechanically induced muscle regeneration, Proc Natl Acad Sci USA. 2016 Feb 9;113(6):1534-9. doi: 10.1073/pnas beschreibt die mechanische Verformung eines Ferrogels mit magnetischen Partikeln durch Anlegen eines magnetischen Feldes. Im Unterscheid zur Erfindung ist es ein ein-Phasen Material ohne Ausrichtung und ohne Stützstruktur. Zur Erläuterung der Erfindung wird aber vollinhaltlich auf diese Veröffentlichung Bezug genommen.

Bei einem Verfahren zur Herstellung eines derartigen Biomaterials wird der zweite Materialanteil eines Implantats zur Regeneration eines spezifischen Gewebes so angepasst, dass sich die für die Regeneration dieses Gewebes benötigten Dehnungen im ersten Materialanteil im Körper ergeben und ein Kollabieren des ersten Materialanteils verhindert wird.

Hierzu werden zunächst die Steifigkeit des intakten Materials und die dort wirkenden mechanischen Kräfte aus der Literatur entnommen. Weiterhin werden aus der Literatur Werte der Gewebedehnung entnommen, die für einen Regenerationsprozess besonders förderlich sind. Mit Hilfe einer 3D Konstruktionssoftware (CAD) werden Einheitszellen entworfen, welche die Steifigkeit in den unterschiedlichen Raumrichtungen auf die nach Implantation auftretenden Kräfte abstimmt (z.B. größte Steifigkeit in Hauptbelastungsrichtung). Die maximale Steifigkeit des Materials muss dabei gleich oder unterhalb der Steifigkeit des intakten Gewebes liegen, um einen mechanischen Reiz für die Regeneration nicht zu blockieren. Im Laufe des Prozesses werden unterschiedliche Designs der Einheitszelle durch Verschieben/Rotieren/Ergänzen/Löschen einzelner Materialstege mittels Computersimulationen basierend auf Finiter Elemente Methode auf ihre mechanischen Eigenschaften getestet. Hierüber werden die im Material auftretenden Dehnungen in Abhängigkeit der wirkenden Kräfte ermittelt. Es wird das Design ausgewählt, welches die Kriterien bezüglich Steifigkeit/Dehnung in den unterschiedlichen Raumrichtungen erfüllt und gleichzeitig die höchst möglich realisierbare Porosität aufweist.

Mehrere Referenzbeispiele sind in den Zeichnungen dargestellt und werden im Folgenden näher erläutert. In diesen Referenzbeispielen ist der zweite Materialanteil nicht wie bei einem Endoskelett integraler und vom ersten Materialanteil umgebener Teil des Biomaterials. Es zeigt
- Figur 1: eine dreidimensionale Ansicht eines offen porösen, bioresorbierbaren ersten Materialanteils,
- Figur 2: eine dreidimensionale Ansicht eines zweiten Materialanteils, der steifer ist als der erste Materialanteil,
- Figur 3: eine dreidimensionale Ansicht einer Kombination aus erstem und zweitem Materialanteil,
- Figur 4: eine dreidimensionale Ansicht einer Kombination aus erstem und zweitem Materialanteil mit unterschiedlichen Strukturen,
- Figur 5: eine Ansicht zur Einbindung der in Figur 3 gezeigten Kombination in einen Gewebebereich,
- Figur 6: eine dreidimensionale Ansicht eines Mechanohybridscaffolds,
- Figur 7: einen Schnitt durch den in Figur 6 gezeigten Mechanohybridscaffold,
- Figur 8: eine Seitenansicht des in Figur 6 gezeigten komprimierten Mechanohybridscaffold,
- Figur 9: zwei Ansichten eines Strukturelements der zweiten Materialkomponente als Basis für Computersimulationen,
- Figur 10: die unteren Grenzwerte der relativen Struktursteifigkeit des in Figur 9 gezeigten Strukturelements als Tabelle,
- Figur 11: einen reinen Kollagenscaffold (a, 1) und einen Mechanohybridscaffold (a, 2) nach Zuschneiden der Prototypen für die mechanische Testung und eine mittels SLM hergestellte Stützstruktur vor Einbringen in den Mechanohybridscaffold (b),
- Figur 12: Bilder des reinen Kollagenscaffolds (a) und des Mechanohybridscaffolds (b) nach Benetzung mit wässriger Pufferlösung sowie das Ergebnis der mechanischen Kompressionstestung (c) und
- Figur 13: die Struktursteifigkeit über der Porosität.

Der in der Figur 1 gezeigte erste Materialanteil hat eine gerichtete Porenstruktur. Diese dient als Leitstruktur und stellt gleichzeitig das eigentliche Zellsubstrat mit optimierten mechanischen und strukturellen Eigenschaften (z.B. Porendurchmesser) und damit die Materialkomponente zur Induktion der Regenerationsprozesse dar. Die mechanischen Eigenschaften sind für die zellulären Prozesse der Migration, Matrixformation und Differenzierung optimiert (intrinsische biomechanische und strukturelle Signale).

Der in der Figur 2 gezeigte zweite Materialanteil (sekundäre Struktur) ist in der gezeigten Realisierung die, ebenfalls strukturell anisotrope, lasttragende Stützstruktur. Sie hat keinen direkten Einfluss auf die zellulären Vorgänge. Allerdings wird durch die höhere Steifigkeit dieser Komponente die makroskopische Integrität des ersten Materialanteils sichergestellt. Die stabile Struktur realisiert eine Aufspann-Funktion und verhindert den Kollaps des als innere Leitstruktur dienenden ersten Materialanteils, während der Gewebeformations- und Umbauprozesse. Außerdem werden über die Steifigkeit des zweiten Materialanteils die mechanischen Dehnungen eingestellt, die aufgrund der lokal wirkenden muskuloskeletalen Kräfte im ersten Materialanteil entstehen. Es werden Dehnungswerte eingestellt, welche die Differenzierung in die gewünschten Gewebetypen (z.B. Knorpel oder Knochen) unterstützen.

Der in Figur 3 gezeigte Mechanohybridscaffold stellt eine dreidimensionale Kombination aus erstem und zweitem Materialanteil dar und Figur 4 eine Ausführungsform, bei der die unterschiedlichen Anforderungen an die Neugewebebildung (Knorpel gegenüber Knochen) durch zwei Bereiche unterschiedlicher Eigenschaften realisiert werden. Hier ist die Dehnbarkeit entsprechend den zellulären Erfordernissen im unteren Bereich des Scaffolds für die knöcherne Regeneration kleiner als im oberen Bereich für die Knorpelregeneration.

In Figur 5 ist eine Ansicht zur Einbindung der in Figur 3 gezeigten Kombination in einen Gewebebereich eines Knochen-Knorpeldefekts dargestellt, wobei die Verwendung der Ausführungsform gemäß Figur 4 eine noch vorteilhaftere Variante zur Regeneration der beiden Gewebstypen Knochen bzw. Knorpel ist.

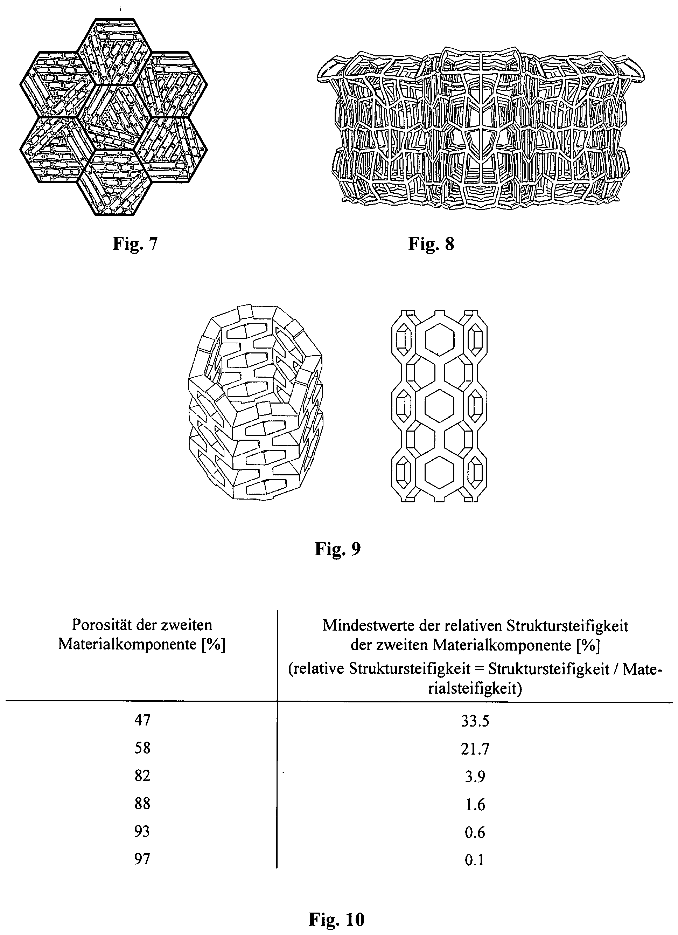

Der in den Figuren 6 bis 8 gezeigte Mechanohybridscaffold besteht aus einem Materialanteil, beispielsweise aus Kollagen und einem zweiten Materialanteil beispielsweise aus PCL. Die Struktur des zweiten Materialanteils wurde bezüglich der mechanischen und strukturellen Eigenschaften optimiert (Anisotropie, definierte Steifigkeit, hohe Porosität, keine Streben mit Ausrichtung senkrecht zur Längsrichtung/Porenrichtung). Die hierarchische Struktur ist durch Vervielfältigung von Einheitszellen aus erster und zweiter Materialkomponente entstanden. Die in Figur 8 gezeigte Verformung bei Belastung wurde mittels Finite Elemente Methode für den Designprozess simuliert, um die gewünschten vorteilhaften Dehnungen zu erzielen.

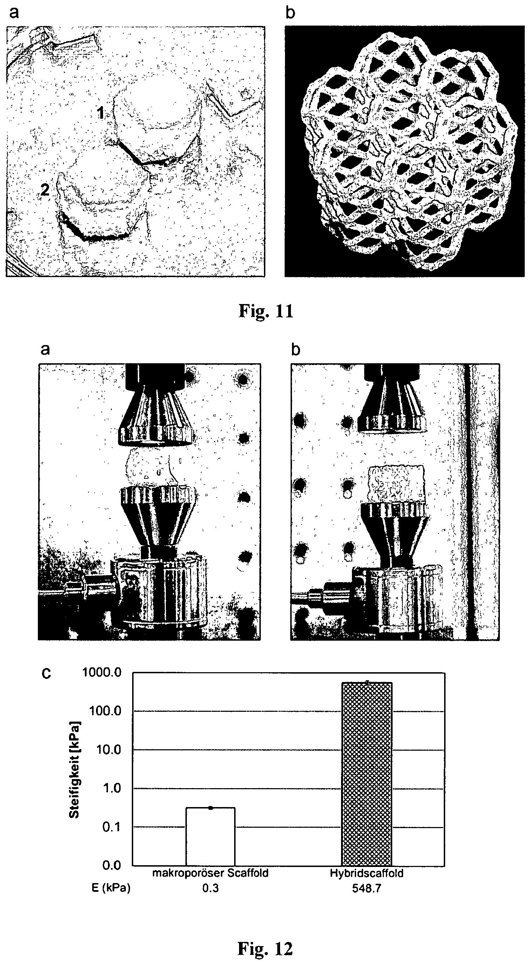

In einem Referenzbeispiel wurde als erster Materialanteil ein weicher Kollagenscaffold mit senkrecht gerichteten Poren mit einer 3D gedruckten Stützstruktur als zweitem Materialanteil kombiniert. Die Stützstruktur wurde aus Polyamid (PA) mittels SLM (Selective Laser Melting) hergestellt. Der Durchmesser der Poren in der Stützstruktur (d.h. der Durchmesser einer Wabe des zweiten Materialanteils) war hierbei ca. 50x größer als der Durchmesser der Poren im ersten Materialanteil, dem Kollagenscaffold. Das Mechanohybridscaffold (Figur 11 a, Scaffold 2) ist durch Herstellung eines sehr weichen Kollagenscaffolds mit gerichteten Poren innerhalb der Stützstruktur (Figur 11 b) hergestellt worden.

Während das reine Kollagenscaffold nach Benetzen mit wässriger Lösung (Phosphatpuffer) kollabiert und damit seine äußere Form verändert (Figur 12a) zeigt das Mechanohybridscaffold als Zeichen der Aufspannfunktion der Sekundärstruktur (=Skelett!!) keine Veränderung der äußeren Geometrie (Figur 12b). Die Figur 12 zeigt somit die mechanische Stabilisierung eines Kollagenscaffolds durch Einbringen einer 3D-gedruckten Stützstruktur aus PA zur Erzeugung eines Mechanohybridscaffolds. Die Bilder der Figur 12 zeigen den reinen Kollagenscaffold (a) und den Mechanohybridscaffold (b) nach Benetzung mit wässriger Pufferlösung. Das Ergebnis der mechanischen Kompressionstestung zeigt den mehr als 1000-fachen Anstieg der Scaffoldsteifigkeit durch Einbringen der Stützstruktur (c). Das Elastizitätsmodul des reinen Kollagenscaffolds und des Kollagen/PA Hybridscaffolds wurde mittels monoaxialer mechanischer Kompressionstestung in einem offenen Reservoir mit wässriger Lösung bestimmt ("unconfined compression test"). Das Elastizitätsmodul E wurde im linearen Bereich der Spannungs-Dehnungs-Kurve analysiert. Die mechanische Testung belegte eine Erhöhung der makroskopischen Steifigkeit von E=0.3(±0.1) kPa (reines Kollagenscaffold) auf 550(±64) kP (Mechanohybridscaffold), also um mehr als das 1000-fache (siehe Figur 12c).

## Patentansprüche

1. Biomaterial, insbesondere für die Geweberegeneration, mit einem offen porösen, bioresorbierbaren ersten Materialanteil und einem zweiten Materialanteil, der steifer ist als der erste Materialanteil und eine lasttragende Stützstruktur bildet, wobei der Volumenanteil des steiferen Materials weniger als 30 %, des Gesamtvolumens des Biomaterials beträgt, *wobei das Gesamtvolumen aus dem Volumen des Biomaterials und dem Volumen vom Biomaterial umschlossener Hohlräume besteht,* die Struktursteifigkeit des zweiten Materialanteils mindestens 10 Mal höher ist als die des ersten Materialanteils, *und der zweite Materialanteil wie bei einem Endoskelett integraler und vom ersten Materialanteil umgebener Teil des Biomaterials ist, sodass* der erste Materialanteil in den zweiten Materialanteil formschlüssig strukturell integriert ist.

2. Biomaterial nach Anspruch 1, ***dadurch gekennzeichnet, dass*** mindestens ein Materialanteil elastisch verformbar ist.

3. Biomaterial nach einem der vorhergehenden Ansprüche, ***dadurch gekennzeichnet, dass*** beide Materialanteile elastisch verformbar sind.

4. Biomaterial nach einem der vorhergehenden Ansprüche, ***dadurch gekennzeichnet, dass*** der Volumenanteil des steiferen Materials weniger als 25 % und bevorzugt weniger als 20 % des Gesamtvolumens des Biomaterials beträgt.

5. Biomaterial nach einem der vorhergehenden Ansprüche, ***dadurch gekennzeichnet, dass*** die Struktursteifigkeit des zweiten Materialanteils 100 Mal und bevorzugt 1000 Mal höher ist als die des ersten Materialanteils.

6. Biomaterial nach einem der vorhergehenden Ansprüche, ***dadurch gekennzeichnet, dass*** die Strukturierung von erstem und zweitem Materialanteil hierarchisch ist.

7. Biomaterial nach einem der vorhergehenden Ansprüche, ***dadurch gekennzeichnet, dass*** mindestens ein Materialanteil und vorzugsweise beide Materialanteile gerichtete Strukturen aufweisen.

8. Biomaterial nach einem der vorhergehenden Ansprüche, ***dadurch gekennzeichnet, dass*** der erste und der zweite Materialanteil Bereiche mit unterschiedlichen gerichteten oder ungerichteten Strukturen aufweisen.

9. Biomaterial nach einem der vorhergehenden Ansprüche, ***dadurch gekennzeichnet, dass*** die Struktur mindestens eines Materialanteils und vorzugsweise beider Materialanteile als repetitives Muster mit Struktursymmetrien aufgebaut ist.

10. Biomaterial nach einem der vorhergehenden Ansprüche, ***dadurch gekennzeichnet, dass*** die Struktur mindestens eines Materialanteils und vorzugsweise beider Materialanteile Stoffe, Elemente oder Strukturen enthält, welche mittels bildgebenden Verfahren darstellbar sind.

11. Biomaterial nach einem der vorhergehenden Ansprüche, ***dadurch gekennzeichnet, dass*** mindestens ein Materialanteil durch einen externen Stimulus zur Regenerationsförderung verformbar ist.

12. Biomaterial nach einem der vorhergehenden Ansprüche, ***dadurch gekennzeichnet, dass*** die Steifigkeit des zweiten Materialanteils in der Richtung am höchsten ist, in der nach Implantation in ein Gewebe die größten Kräfte auf das Material wirken (Hauptbelastungsrichtung).

13. Biomaterial nach einem der vorhergehenden Ansprüche, ***dadurch gekennzeichnet, dass*** die Architektur des zweiten Materialanteils so optimiert ist, dass zur Realisierung einer bestimmten makroskopischen Materialsteifigkeit eine möglichst hohe Porosität erreicht werden kann.

14. Biomaterial nach einem der vorhergehenden Ansprüche, ***dadurch gekennzeichnet, dass*** sich die mittleren Durchmesser der Strukturelemente des zweiten Materialanteils innerhalb einer Grundgeometrie/ Einheitszelle um weniger als einen Faktor zwei unterscheiden.

## Claims

1. A biomaterial, in particular for tissue regeneration, comprising an openly porous, bioabsorbable first material fraction and a second material fraction that is stiffer than the first material fraction and forms a load-bearing support structure, wherein the volume fraction of the stiffer material is less than 30 % of the total volume of the biomaterial, wherein the total volume consists of the volume of the biomaterial and the volume of voids enclosed by the biomaterial, the structural stiffness of the second material fraction is at least 10 times higher than that of the first material fraction, and the second material fraction is an integral part of the biomaterial surrounded by the first material fraction, as in an endoskeleton, such that the first material fraction is structurally integrated into the second material fraction in a form-fitting manner.

2. The biomaterial according to claim 1, ***characterised in that*** at least one material fraction is elastically deformable.

3. The biomaterial according to any one of the preceding claims, ***characterised in that*** both material fractions are elastically deformable.

4. The biomaterial according to any one of the preceding claims, ***characterised in that*** the volume fraction of the stiffer material is less than 25 % and preferably less than 20 % of the total volume of the biomaterial.

5. The biomaterial according to any one of the preceding claims, ***characterised in that*** the structural stiffness of the second material fraction is 100 times and preferably 1000 times higher than that of the first material fraction.

6. The biomaterial according to any one of the preceding claims, ***characterised in that*** the structuring of first and second material fraction is hierarchical.

7. The biomaterial according to any one of the preceding claims, ***characterised in that*** at least one material fraction and preferably both material fractions have directed structures.

8. The biomaterial according to any one of the preceding claims, ***characterised in that*** the first and the second material fraction have regions with different directed or undirected structures.

9. The biomaterial according to any one of the preceding claims, ***characterised in that*** the structure of at least one material fraction and preferably both material fractions is constructed as a repetitive pattern having structural symmetries.

10. The biomaterial according to any one of the preceding claims, ***characterised in that*** the structure of at least one material fraction and preferably both material fractions contains substances, elements or structures which can be displayed by means of imaging methods.

11. The biomaterial according to any one of the preceding claims, ***characterised in that*** at least one material fraction is deformable by an external stimulus for regeneration promotion.

12. The biomaterial according to any one of the preceding claims, ***characterised in that*** the stiffness of the second material fraction is highest in the direction in which the largest forces act on the material after implantation in a tissue (main load direction).

13. The biomaterial according to any one of the preceding claims, ***characterised in that*** the architecture of the second material fraction is optimised so that, to realise a certain macroscopic material stiffness, a highest possible porosity can be achieved.

14. The biomaterial according to any one of the preceding claims, ***characterised in that*** the mean diameters of the structural elements of the second material fraction within a basic geometry/unit cell differ by less than a factor of two.

## Revendications

1. Matériau biologique, destiné notamment à la régénération tissulaire, comportant une première proportion de matériau biorésorbable à pores ouverts et une deuxième proportion de matériau qui est plus rigide que la première proportion de matériau et forme une structure de soutien supportant les charges, la proportion volumique du matériau plus rigide étant inférieure à 30 % par rapport au volume total dudit matériau biologique, le volume total étant constitué du volume dudit matériau biologique et du volume des cavités entourées par le matériau biologique, la rigidité structurelle de la deuxième proportion de matériau étant au moins 10 fois supérieure à celle de la première proportion de matériau, et la deuxième proportion de matériau faisant partie intégrante dudit matériau biologique tout en étant entourée, à la manière d'un endosquelette, par la première proportion de matériau, faisant en sorte que la première proportion de matériau soit intégrée structurellement à la deuxième proportion de matériau par complémentarité de forme.

2. Matériau biologique selon la revendication 1, ***caractérisé en ce qu**'*au moins une proportion de matériau peut subir une déformation élastique.

3. Matériau biologique selon l'une des revendications précédentes, ***caractérisé en ce que*** les deux proportions de matériau peuvent subir une déformation élastique.

4. Matériau biologique selon l'une des revendications précédentes, ***caractérisé en ce que*** la proportion volumique du matériau plus rigide représente moins de 25 %, et préférentiellement moins de 20 %, du volume total dudit matériau biologique.

5. Matériau biologique selon l'une des revendications précédentes, ***caractérisé en ce que*** la deuxième proportion de matériau présente une rigidité structurelle qui est 100 fois supérieure, et préférentiellement 1000 fois supérieure, à celle de la première proportion de matériau.

6. Matériau biologique selon l'une des revendications précédentes, ***caractérisé en ce que*** la première et la deuxième proportions de matériau présentent une structuration hiérarchique.

7. Matériau biologique selon l'une des revendications précédentes, ***caractérisé en ce que*** l'une des proportions de matériau, et préférentiellement les deux proportions de matériau, présentent des structures orientées.

8. Matériau biologique selon l'une des revendications précédentes, ***caractérisé en ce que*** la première et la deuxième proportions de matériau présentent des zones comportant différentes structures orientées ou non-orientées.

9. Matériau biologique selon l'une des revendications précédentes, ***caractérisé en ce que*** la structure d'au moins une des proportion de matériau, et préférentiellement des deux proportions de matériau, est réalisée sous forme d'un schéma répétitif présentant des symétries structurelles.

10. Matériau biologique selon l'une des revendications précédentes, ***caractérisé en ce que*** ladite structure d'au moins une des proportions de matériau, et préférentiellement des deux proportions de matériau, contient des éléments ou structures pouvant être représentés au moyen de méthodes d'imagerie.

11. Matériau biologique selon l'une des revendications précédentes, ***caractérisé en ce qu***'au moins une proportion de matériau peut être déformée par un stimulus externe afin de favoriser la régénération.

12. Matériau biologique selon l'une des revendications précédentes, ***caractérisé en ce*** la deuxième proportion de matériau présente sa plus forte rigidité dans la direction selon laquelle ledit matériau subira, après l'implantation dans un tissu, les forces les plus importantes (direction de charge principale).

13. Matériau biologique selon l'une des revendications précédentes, ***caractérisé en ce que*** l'architecture de la deuxième proportion de matériau est optimisée de manière à ce qu'elle permette d'atteindre une porosité la plus élevée possible afin de réaliser une certaine rigidité de matériau sur le plan macroscopique.

14. Matériau biologique selon l'une des revendications précédentes, ***caractérisé en ce que*** les diamètres moyens des éléments structurels de la deuxième proportion de matériau varient, au sein d'une géométrie de base / cellule unitaire, d'un facteur inférieur à deux.
